# EUROPEAN PATENT APPLICATION

(11) **EP 4 538 363 A1**
(43) Date of publication of application: **16.04.2025**
(21) Application number: 23824177.2
(22) Date of filing: 12.06.2023
(51) Int. Cl.: C12N 5/00, C12N 5/0775, C12N 5/077, A61P 19/00

(54) **EXTRACELLULAR VESICLE MASS PRODUCTION METHOD USING CELL SPHEROID EMBEDDED WITH MICROPARTICLE HAVING DIFFERENTIATION STIMULATING FACTOR SUPPORTED THEREIN**

(30) Priority: 13.06.2022 KR 20220071587
(71) Applicant: Dongguk University Industry-Academic Cooperation Foundation, Seoul 04620 (KR)
(72) Inventor: LEE, Soo-Hong, Hanam-si Gyeonggi-do 12912 (KR); ARAI, Yoshie, Goyang-si Gyeonggi-do 10325 (KR); CHO, Woong-Jin, Paju-si Gyeonggi-do 10923 (KR); PARK, Hyo-Eun, Uijeongbu-si Gyeonggi-do 11718 (KR)
(74) Representative: Mewburn Ellis LLP
(86) International application number: PCT/KR2023/008025
(87) International publication number: WO 2023/243960

(57) **Abstract**

The present invention relates to an extracellular vesicle mass production method using a cell spheroid embedded with a microparticle having a differentiation stimulating factor supported therein, and the extracellular vesicle obtained through the production method has the effect of regenerating a tissue, in particular, a cartilage tissue, and, thus, can be used to treat diseases related to tissue regeneration and cartilage by utilizing such effect.

## Description

### Technical Field

The present disclosure provides an extracellular vesicle production method using a cell spheroid embedded with differentiation stimulating factor-loaded microparticles.

### Background Art

The study on nano-sized extracellular vesicles has received a lot of attention as it has been discovered that the paracrine effect, one of the important causative mechanisms considered important in tissue regeneration and disease treatment using cells, is derived from extracellular vesicles.

Recently discovered functions of extracellular vesicles include tissue regeneration, immunomodulation, drug delivery, and cancer treatment, and research and development of extracellular vesicles for diagnosis are being conducted as well.

However, natural extracellular vesicles extracted from cells show low yield efficiency as well as low tissue regenerative functions. Cell culture using a three-dimensional (3D) environment bears a high possibility of producing a large quantity of extracellular vesicles that are produced by cells due to easiness in cell culture in bulk, and it is also possible to improve histological functions of cells by mimicking in vivo environment in 3D. For example, cell spheroids show higher expression of cartilage-related genes than cells cultured in a two-dimensional (2D) environment.

The cell spheroid culture method, which is a conventional 3D cell culture method, involves formation of a necrosis zone at the center of the cell to cause a poor supply of nutrients to the center, making it infeasible to culture cells healthy for a long time.

Therefore, there is a need for a technology to improve the production efficiency of extracellular vesicles and increase tissue regenerative functionality by developing a 3D cell culture method that may increase the production efficiency of extracellular vesicles and tissue regenerative functions of cells.

### Disclosure of the Invention

### Technical Goals

An object of the present disclosure is to provide an extracellular vesicle production method, including: preparing a microparticle from one or more polymers selected from the group consisting of polylactic-co-glycolic acid (PLGA), gelatin, arginine, and hyaluronic acid; loading a differentiation stimulating factor in the microparticle; adding the microparticles loaded with the differentiation stimulating factor to a cell suspension and then dispensing them into a cell adhesion-inhibited microwell to prepare a cell spheroid; and obtaining an extracellular vesicle from the cell spheroid.

Another object of the present disclosure is to provide a pharmaceutical composition for tissue regeneration, including the extracellular vesicle obtained by the production method as an active ingredient.

Another object of the present disclosure is to provide a pharmaceutical composition for preventing or treating a cartilage disease, including the extracellular vesicle obtained by the production method as an active ingredient.

Another object of the present disclosure is to provide a health functional food composition for improving cartilage functions, including the extracellular vesicle obtained by the production method as an active ingredient.

### Technical Solutions

To achieve the above objects, the present disclosure provides an extracellular vesicle production method, including: preparing a microparticle from one or more polymers selected from the group consisting of polylactic-co-glycolic acid (PLGA), gelatin, arginine, and hyaluronic acid; loading a differentiation stimulating factor in the microparticle; adding the microparticles loaded with the differentiation stimulating factor to a cell suspension and then dispensing them into a cell adhesion-inhibited microwell to prepare a cell spheroid; and obtaining an extracellular vesicle from the cell spheroid.

In addition, the present disclosure provides a pharmaceutical composition for tissue regeneration, including the extracellular vesicle obtained by the production method as an active ingredient.

In addition, the present disclosure provides a pharmaceutical composition for preventing or treating a cartilage disease, including the extracellular vesicle obtained by the production method as an active ingredient.

In addition, the present disclosure provides a health functional food composition for improving cartilage functions, including the extracellular vesicle obtained by the production method as an active ingredient.

### Advantageous Effects

The present disclosure relates to an extracellular vesicle mass production method using a cell spheroid embedded with differentiation stimulating factor-loaded microparticles, wherein an extracellular vesicle obtained through the production method has tissue regenerability, especially an effect of regenerating cartilage tissues and thus may be used for tissue regeneration and treatment of cartilage-related diseases.

### Brief Description of Drawings

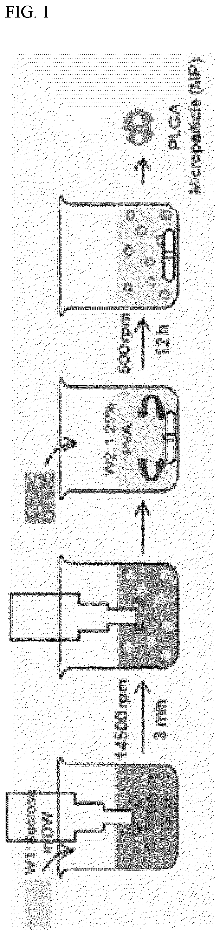

FIG. 1 shows a method of producing a microparticle.
FIG. 2 shows results of identifying a shape and size of a prepared microparticle.

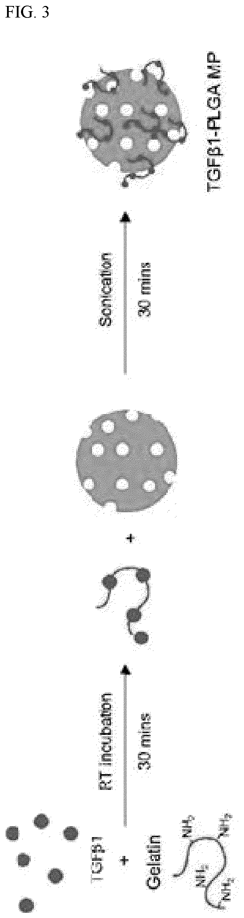

FIG. 3 shows a method of loading a differentiation stimulating factor into a microparticle.
FIG. 4 shows a result of identifying a TGF-β1 loading efficiency in a microparticle.
FIG. 5 shows a result of identifying TGF-β1 release patterns from a microparticle loaded with TGF-β1.
FIG. 6 shows results of microscopic observation of mesenchymal stem cells.
FIG. 7 shows results of microscopic observation of prepared cell spheroids.

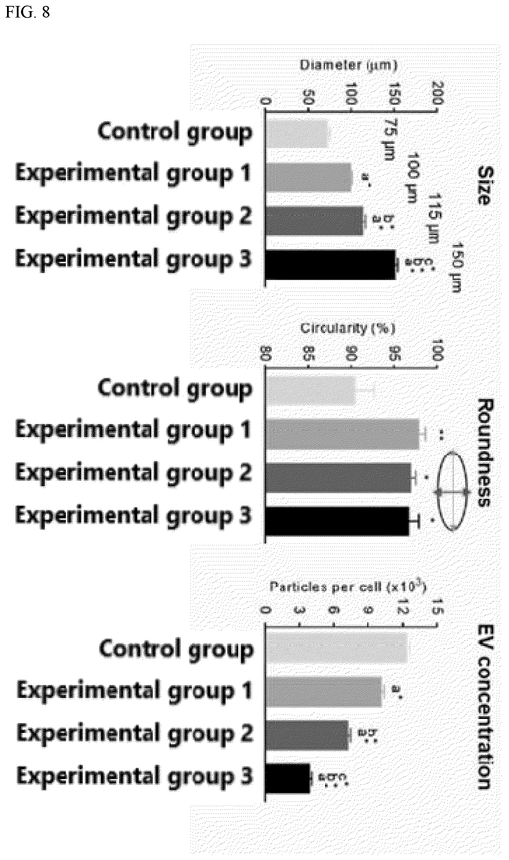

FIG. 8 shows results of determining a size and roundness of a cell spheroid, as well as an amount of extracellular vesicles released from the cell spheroid. In a graph of cell spheroid size, a* indicates a significant increase compared to a control group. b* indicates a significant increase compared to an experimental group 1. c* indicates a significant increase compared to an experimental group 2. In a graph for a yield of extracellular vesicles, a* indicates a significant decrease compared to the control group. b* indicates a significant decrease compared to the experimental group 1. c* indicates a significant decrease compared to the experimental group 2.
FIG. 9 shows results of microscopic observation of cell spheroids embedded with a differentiation stimulating factor-loaded microparticle.
FIG. 10 shows a result of identifying an amount of extracellular vesicles produced during induction of cartilage differentiation.
FIG. 11 shows a result of identifying protein expression of cartilage-related genes present in extracellular vesicles.
FIG. 12 shows results of quantifying protein expression of cartilage-related genes present in extracellular vesicles.

### Best Mode for Carrying Out the Invention

Hereinafter, the present disclosure will be described in more detail.

The present disclosure provides a method of producing an extracellular vesicle, including: preparing a microparticle from one or more polymers selected from the group consisting of polylactic-co-glycolic acid (PLGA), gelatin, arginine, and hyaluronic acid; loading a differentiation stimulating factor in the microparticle; adding the microparticles loaded with the differentiation stimulating factor to a cell suspension and then dispensing them into a cell adhesion-inhibited microwell to prepare a cell spheroid; and obtaining an extracellular vesicle from the cell spheroid.

The preparing of the microparticle may include mixing a sugar solution and a polymer solution to obtain an emulsion solution; and adding the emulsion solution dropwise to a polyvinyl alcohol solution followed by stirring and then evaporating a solvent present in the solution.

An average diameter of the microparticles may be 15 µm to 22 µm.

The loading may include adding the microparticle to a solution in an ionically bonded state having gelatin mixed in the differentiation stimulating factor and reacting them.

The differentiation stimulating factor may be selected from the group consisting of TGF-β1, TGF-β3, BMP-2, BMP4, and bile acids, but is not limited to.

An average loading efficiency of the differentiation stimulating factor may be 50% to 60%.

The cell suspension may be selected from the group consisting of human adipose-derived mesenchymal stem cells, human bone marrow-derived mesenchymal stem cells, human hematopoietic stem cells, human chondrocytes, and human induced pluripotent stem cell-derived mesenchymal stem cells, but is not limited to.

An average diameter of the cell spheroid may be 75 µm to 115 µm. In addition, the cell spheroid may have increased expression of COL2 and ACAN proteins.

The extracellular vesicle may be one which is obtained by adding a cartilage differentiation-inducible culture medium into the cell spheroid followed by culture. In addition, the extracellular vesicle may be a cartilaginous functional extracellular vesicle.

In addition, the present disclosure provides a pharmaceutical composition for tissue regeneration, including the extracellular vesicle obtained by the production method as an active ingredient.

The tissue regeneration may be for the regeneration of cartilage tissues.

In another embodiment of the present disclosure, the pharmaceutical composition may further include one or more additives selected from the group consisting of appropriate carriers, excipients, disintegrators, sweeteners, coating agents, leavening agents, lubricants, glydents, flavoring agents, antioxidants, buffers, bacteriostatic agents, diluents, dispersants, surfactants, binders, and lubricants that are commonly used in the manufacture of pharmaceutical compositions. Specifically, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearate and mineral oil may be used as the carriers, excipients, and diluents, solid preparations for oral administration may include tablets, pills, acids, granules, and capsules, and these solid preparations may be prepared by mixing, in the composition, at least one or more excipients, for example, starch, calcium carbonate, sucrose or lactose, and gelatin. In addition to simple excipients, lubricants such as magnesium stearate and talc may also be used. Oral liquid preparations may include suspensions, liquids, emulsions, and syrups, various excipients, such as humectants, sweeteners, fragrances, and preservatives may be included in addition to the simple diluents that are commonly used, such as water and liquid paraffin. Preparations for parenteral administration include sterile aqueous solutions, non-aqueous solvents, suspensions, emulsions, lyophilized agents, and suppositories. For non-aqueous solvents and suspensions, propylene glycol, polyethylene glycol, vegetable oils such as olive oil, and injectable esters such as ethyl oleate may be used. As for base materials of suppositories, witepsol, macrogol, tween 61, cacao butter, laurin fat, and glycerogelatin may be used. According to one embodiment of the present disclosure, the pharmaceutical composition may be administered to a subject in a conventional manner through intravenous, intraarterial, intraperitoneal, intramuscular, intraarterial, intraperitoneal, intrasternal, transdermal, intranasal, inhalation, topical, rectal, oral, intraocular, or intradermal routes. The dosage of the active ingredient according to the present disclosure may vary depending on the condition and weight of the subject, the type and degree of disease, the form of the drug, and the route and duration of administration and be appropriately selected by those skilled in the art, and the daily dose may be 0.01 mg/kg to 200 mg/kg, preferably 0.1 mg/kg to 200 mg/kg, and more preferably 0.1 mg/kg to 100 mg/kg. The administration may be conducted once a day or divided into several doses, which does not limit the scope of the present disclosure.

The tissues whose regeneration is facilitated by the composition of the present disclosure are not particularly limited but include several tissues in the whole body. Involved tissues include, but are not limited to, for example, liver, pancreas, kidneys, large intestine, lungs, spleen, heart, bone marrow, vocal cords, skin, peritoneum, eyes, and blood vessels.

The composition of the present disclosure is conducive to tissue regeneration arising from impaired or grafted tissue. Impaired tissue includes tissues that have undergone tissue destruction, inflammation, necrosis, fibrotic surgical invasion, and organ failure. The timing of administration of the composition of the present disclosure is not particularly limited, but it is desirable to administer it within 4 days from the starting date of the disability or transplantation or within 1 day from the starting date of the disability or implantation.

Tissue regeneration by the composition of the present disclosure may be accompanied by differentiation and/or proliferation of stem cells. In addition, tissue regeneration by the composition of the present disclosure may be accompanied by the proliferation of actual cells in the tissues. Differentiation of stem cells may be assessed, for example, by the detection of cell markers specific to differentiated cells, and by the detection of the functions derived by differentiated cells. Cell proliferation may be assessed by various known methods, e.g., calculation of the number of living cells over time, measurement of tissue size, volume, or weight, measurement of DNA synthesis, WST-1 method, bromo deoxyuridine (BrdU) method, and 3 H thymidine capture.

The composition of the present disclosure may be used for an individual with tissue regeneration suppressed. A state in which tissue regeneration is suppressed includes, but is not limited to, for example, inflammation, necrosis, fibrosis, organ failure, decreased platelets, genetic abnormalities, and decreased noradrenaline.

It is desirable that the blending amount of the active ingredient in the composition of the present disclosure is an amount that promotes tissue regeneration if the composition is administered. In addition, it is desirable to have a quantity that does not cause adverse effects that exceed the benefits by administration. The amount required may be appropriately determined according to the known or in vitro tests using cultured cells, and in animal models such as mice, rats, dogs, and pigs, and these test methods are well known to those skilled in the art. In the case of promotion of tissue regeneration, evaluation may be made on the recovery of tissue function, weight, and size by biochemical examination, X-ray, ultrasound, MRI, CT, and visual diagnosis using endoscopy. The blending amount of the active ingredient may vary depending on the dosage form of the composition. For example, if several units of the composition are used in a single administration, the blending amount of active ingredient in one unit of the composition may be one over plural of the amount of the active ingredient required for a single administration. The required blending amount may be adjusted appropriately by those skilled in the arts.

In addition, the present disclosure provides a pharmaceutical composition for preventing or treating a cartilage disease, including the extracellular vesicle obtained by the production method as an active ingredient.

In addition, the present disclosure provides a health functional food composition for improving cartilage functions, including the extracellular vesicle obtained by the production method as an active ingredient.

The health functional food may contain various nutritional supplements, vitamins, minerals (electrolytes), flavor agents such as synthetic flavor agents and natural flavor agents, coloring agents and thickeners (cheese and chocolate), pectic acid and salts thereof, alginate and salts thereof, organic acids, protective colloidal thickeners, pH regulators, stabilizers, preservatives, glycerin, alcohol, and carbonatig agents used in carbonated beverages. In addition, it may contain pulp for the manufacture of natural fruit juices, synthetic fruit juices, and vegetable drinks. These ingredients may be used independently or in combination. In addition, the health functional food composition may be in the form of any one of meat, sausage, bread, chocolate, candy, snacks, confectionery, pizza, instant noodles, chewing gum, ice cream, soups, beverages, teas, functional waters, drinks, alcohol, and vitamin complexes. In addition, the health functional food may additionally contain food additives, and the suitability as the "food additive" is determined by the standards and criteria related to corresponding items according to the general rules and general test methods of Korean Food Additives Codex approved by the Ministry of Food and Drug Safety, unless otherwise stipulated. The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents. Here, the content of active ingredients added to the food in the process of manufacturing the health functional food may be appropriately adjusted as needed, and preferably it may be added to be included in an amount of 1 part by weight to 90 parts by weight of 100 parts by weight of the food.

Hereinafter, the present disclosure will be described in more detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

### Modes for Carrying Out the Invention

### <Example 1> Preparation of TGF-β1-loaded PLGA microparticle

### 1. Preparation of PLGA microparticle

After mixing a solution in which 0.72 g of sucrose is dissolved in 0.6 ml of distilled water and a solution in which 50 mg of poly lactic-co-glycolic acid (hereinafter referred to as PLGA) is dissolved in dichloromethane (DCM), emulsification was carried out for 3 minutes at 14,500 rpm using a homogenizer. The emulsified solution was added dropwise to 1.25% polyvinyl alcohol (hereinafter referred to as PVA) dissolved in 50 ml of distilled water and stirred at 500 rpm for 12 hours. During the stirring time, DCM evaporated, and PLGA particles with a size of 100 µm or greater remaining in the 1.25% PVA solution were removed using a 100 µm strainer, followed by centrifugation at 3,000 rpm for 10 minutes to obtain PLGA microparticles. The obtained PLGA particles were treated with 50 ml of distilled water, washing by centrifugation was repeated three times for 10 minutes at 3,000 rpm to obtain the PLGA microparticles, and the pure PLGA microparticles were obtained through the lyophilization process.

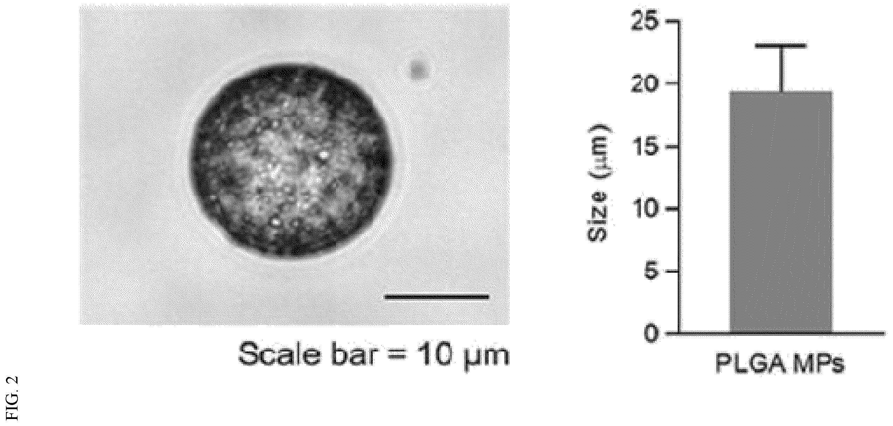

As a result, according to FIG. 2, it was found that a hollow microparticle with small size holes inside the microparticle was produced. As a result of analyzing the size through microscopic images, it was determined that the average diameter of the microparticles is about 19.3 µm.

### 2. Loading of TGF-β1 into PLGA microparticle

After mixing 25 µl of a solution in which 20 mg of gelatin is dissolved in 1 ml of distilled water and a solution in which 16 µg of TGF-β1 is dissolved in 25 µl of distilled water, a reaction was carried out for 30 minutes at room temperature to prepare 50 µl of a solution in a state in which gelatin and TGF-β1 are ionically bonded. After mixing 10 mg of PLGA microparticles with 50 µl of a solution in which gelatin and TGF-β1 are ionically bonded, TGF-β1 was loaded in the PLGA microparticles through immersion for 30 minutes at room temperature with sonication. The PLGA microparticles loaded with TGF-β1 were centrifuged at 3,000 rpm for 10 minutes to obtain PLGA microparticles, followed by three times repetition of a washing process in which 50 ml of distilled water was treated and centrifuged for 10 minutes at 3,000 rpm. After undergoing the lyophilization process, PLGA microparticles loaded with TGF-β1 were finally obtained.

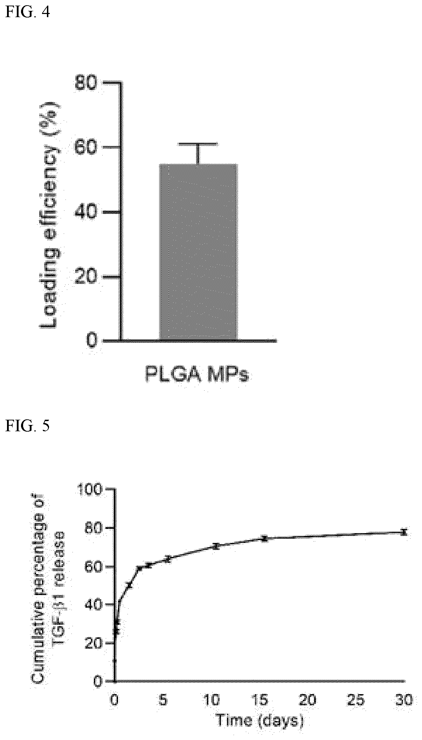

As a result, according to FIG. 4, it was found that the loading efficiency of the prepared TGF-β1-loaded microparticles is about 52.9%. The amount of TGF-β1 loaded was determined by enzyme-linked immunoassay (EIA) after dissolving the prepared microparticles with chloroform. In addition, according to FIG. 5, 0.5 µg of microparticles loaded with TGF-β1 were diluted in 1 ml of phosphate buffer solution, and then the amount of factor released at each time was checked through enzyme-linked immunoassay (EIA). Rapid release was observed within the initial 24 hours, followed by a slow release.

### <Example 2> Identification of efficiency in cell spheroid preparation and extracellular vesicle production

### 1. Preparation of cell spheroids embedded with differentiation stimulating factor-loaded microparticles

The cryopreserved mesenchymal stem cells were dispensed at 1,000,000 cells per 150 mm TCP cell culture dish and then cultured for 7 days in an incubator at 37°C in the presence of 5% CO₂, with replacement of 20 ml of a culture medium (DMEM-Low glucose, 10% fetal bovine serum, 1% penicillin/streptomycin) every 2 days. After 7 days, 3 ml of trypsin per 150 mm TCP cell culture dish was treated at 37°C for 3 minutes to remove the adhering cells and collect a total of 3 million cells to prepare 6 ml of a culture medium suspended with 500,000 cells per 1 ml. The prepared mesenchymal stem cells were used and cultured in a 2D environment or a 3D environment as shown in Table 1.

**TABLE 1**

| Category | Environment | Condition |
|---|---|---|
| Control group | 2D | Single-layer culture |
| Experimental group | 3D | Cell spheroid culture |

The control group cultured in a 2D environment was cultured in an incubator at 37°C in the presence of 5% CO₂ by dispensing 1 ml of cell suspension containing 500,000 cells per 100 mm culture dish into 10 ml of a culture medium (DMEM-Low glucose, 10% fetal bovine serum, 1% penicillin/streptomycin). The experimental group cultured in a 3D environment was treated with 0.5 ml of anti-adhesion solution for 30 minutes per microwell having 389 wells in a size of 600 µm to prepare a microwell with cell adhesion inhibited, and then 1 ml of cell suspension containing 500,000 cells per microwell with cell adhesion inhibited was dispensed to prepare a cell spheroid by culture for 24 hours.

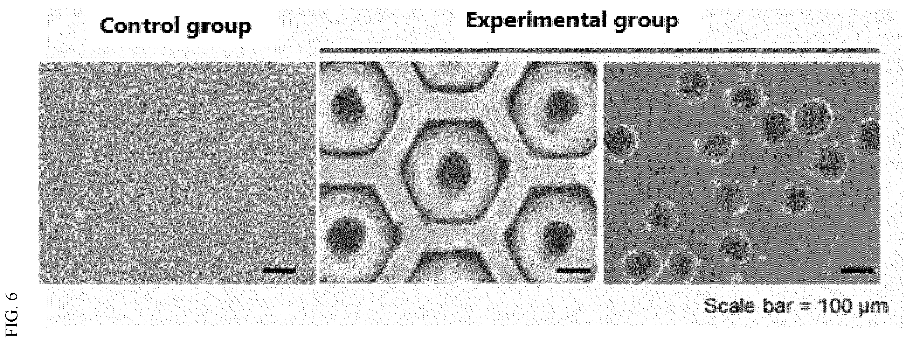

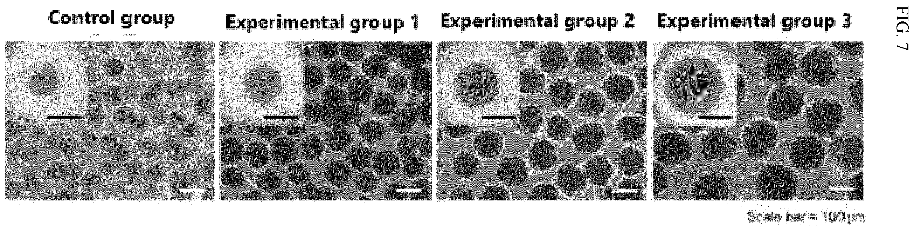

As a result, according to FIG. 6, it was found that, in the control group, mesenchymal stem cells were attached to the bottom of the culture dish and showed an elongated shape. In the experimental group, it was found that mesenchymal stem cells did not attach to the bottom of the culture dish, but formed spheroids in the uniform size in the spherical shape with a cluster of cells. In addition, according to FIG. 7, it was found that cell spheroids of various sizes were prepared by controlling the number of cells contained in a single cell spheroid as shown in Table 2.

**TABLE 2**

| Category | Counts of cells per microwell | Counts of cells per cell spheroid |
|---|---|---|
| Control group | 250,000 | About 643 |
| Experimental group 1 | 500,000 | About 1,285 |
| Experimental group 2 | 1,000,000 | About 2,571 |
| Experimental group 3 | 2,000,000 | About 5,141 |

In addition, according to FIG. 8, it was found that the greater the number of cells that make up a single cell spheroid, the significantly larger the length of the diameter of the cell spheroid. In the case of roundness, a comparison was made on the ratio of short axis to the long axis, determining that almost 100% perfect spherical shape was shown in the experimental group 1, experimental group 2, and experimental group 3 compared to the control group. Extracellular vesicles were obtained after culturing cells with cartilage differentiation induced for 7 days in a serum-free culture medium for 24 hours. The amount of extracellular vesicle obtained was identified using EXOCET (Exosome Quantitation Kit). In the comparative results for the yield of extracellular vesicles, it was found that the smaller the size of the cell spheroids, the higher the yield. The experimental group 1, whose form of the cell spheroid was closest to the sphere with the highest yield of extracellular vesicles, was found to be suitable for the production of extracellular vesicles.

After mixing 0.5 mg of microparticles in 1 ml of cell suspension containing 500,000 cells, they were dispensed into microwells with cell adhesion inhibited and cultured for 24 hours to produce cell spheroids embedded with differentiation stimulating factor-loaded microparticles.

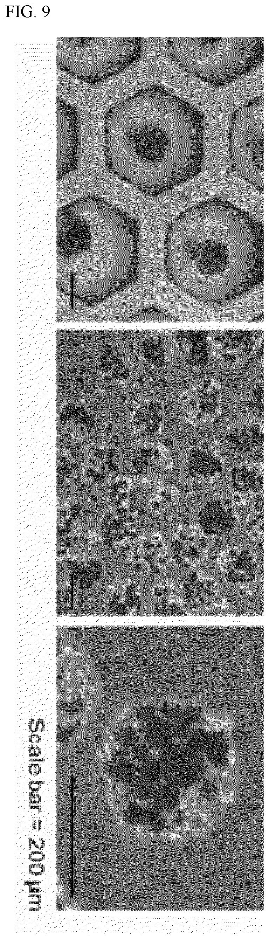

As a result, according to FIG. 9, it may be found that the microparticles are located inside the cell spheroids, forming spheroids of uniform size in the shape of a sphere.

### 2. Production efficiency of extracellular vesicles

As shown in Table 3, extracellular vessels were obtained from mesenchymal stem cells with differentiation induced for 7 days in a 2D environment and a 3D environment, followed by analysis on the production volume.

**TABLE 3**

| Category | Environment | Condition | Differentiation stimulating factor |
|---|---|---|---|
| Control group | 2D | Single-layer culture | Soluble TGF-β1 |
| Experimental group 4 | 3D | Cell spheroid culture | Soluble TGF-β1 |
| Experimental group 5 | 3D | Cell spheroid culture | Microparticle TGF-β1 |

The control group was cultured in an incubator at 37°C in the presence of 5% CO₂ for 7 days, by adding 10 ng of dissolved TGF-β1 to 10 ml of a cartilage differentiation-inducible culture medium (DMEM-High glucose, 10% fetal bovine serum, 1% penicillin/streptomycin, 1× Insulin-Transferrin-Selenium, 50 µg/ml ascorbic acid, 100 nM dexamethason) at 500,000 cells per 100 mm culture dish. The experimental group 4 was cultured for 7 days in an incubator at 37°C in the presence of 5% CO₂, by treating 5 ml of an anti-adhesion solution per 100 mm culture dish for 30 minutes, transferring cell spheroids prepared in one microwell to the culture dish with cell adhesion inhibited, adding 10 ng of dissolved TGF-β1 to 10 ml of a cartilage differentiation-inducible culture medium, and shaking the mixture at 120 rpm in a 3D shaker. The experimental group 5 was cultured for 7 days in an incubator at 37°C in the presence of 5% CO₂, by treating 5 ml of an anti-adhesion solution per 100 mm TCP culture dish for 30 minutes, transferring cell spheroids embedded with TGF-β1-loaded microparticles prepared in a single microwell to the culture dish with cell adhesion inhibited, and shaking at 120 rpm in a 3D shaker along with 10 ml of a cartilage differentiation-inducible culture medium. The culture medium was replaced once every two days. The amount of extracellular vesicle obtained was determined using EXOCET (Exosome Quantitation Kit).

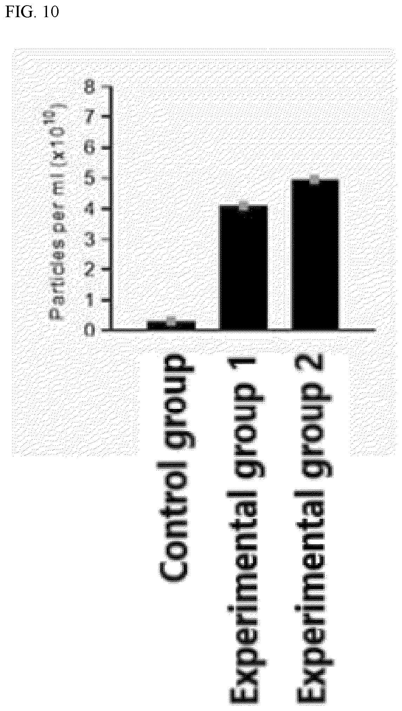

As a result, according to FIG. 10, it was found that the amount of extracellular vesicles obtained was increased in the experimental group 4 and the experimental group 5 compared to the control group. In addition, in the experimental group 2, which is a spheroid embedded with TGF-β1-loaded microparticles, it was observed that the amount of extracellular vesicle obtained increased compared to the experimental group 1 with no microparticles.

From this, it was found that spheroids embedded with TGF-β1-loaded microparticles had the highest yield of extracellular vesicle than spheroids embedded with TGF-β1, indicating that the functionality was enhanced compared to spheroids that are simply embedded with TGF-β1.

### <Example 3> Identification of cartilage characteristics of extracellular vesicles of mesenchymal stem cells in a 3D culture environment

As shown in Table 3, extracellular vesicles were obtained from mesenchymal stem cells with induced differentiation for 7 days in a 2D environment and a 3D environment, and the expression of cartilage-related genes was checked using WES system (ProteinSimple), an automated protein expression analyzer.

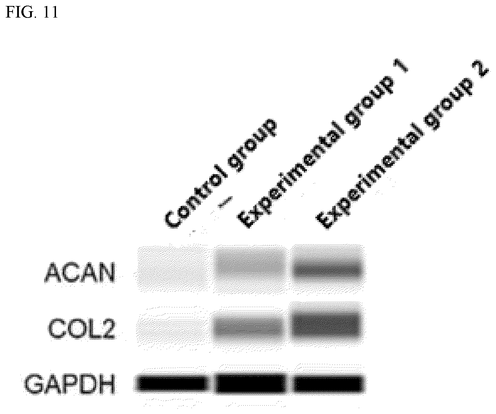

As a result, according to FIG. 11, it was found that the cartilage-related genes of type 2 collagen (COL2) and ACAN proteins present inside the extracellular vesicle were expressed.

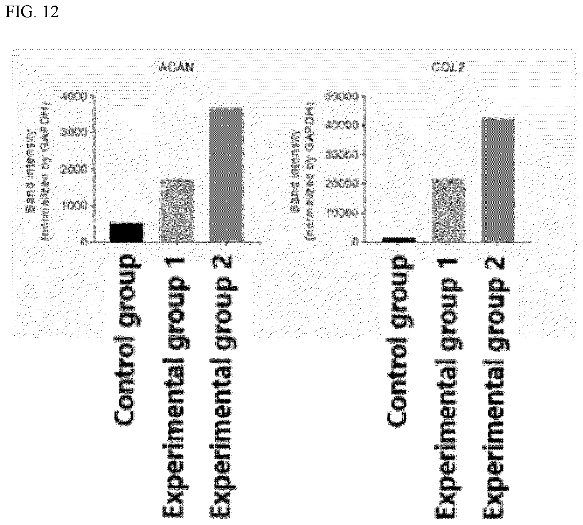

In addition, according to FIG. 12, compared to the cell control group with differentiation induced by adding TGF-β1 in a 2D culture environment, it was found that, by adding TGF-β1 in the 3D environment, COL2 and ACAN protein expression increased in the experimental group 4 with differentiation induced cell spheroids and the experimental group 5 with the cell spheroid embedded with TGF-β1-loaded microparticles in the 3D culture environment. In addition, it was found that COL2 and ACAN protein expression increased in experimental group 5 compared to experimental group 4.

The foregoing description of the present disclosure is for illustrative purposes only, and it will be understood by those skilled in the art to which the present disclosure pertains that the present disclosure may be easily modified into other specific forms without changing the technical idea or essential features of the present disclosure. Therefore, it should be appreciated that the foregoing embodiments are illustrative in all respects but not limiting.

The scope of the present disclosure is represented by the appended claims, and the meaning and scope of the claims and any altered or modified form derived from the concept of equivalence should be construed as being included within the scope of the disclosure.

## Claims

1. A method of producing an extracellular vesicle, the method comprising:
(1) preparing a microparticle from one or more polymers selected from the group consisting of polylactic-co-glycolic acid (PLGA), gelatin, arginine, and hyaluronic acid;
(2) loading a differentiation stimulating factor in the microparticle of step (1);
(3) adding the microparticle loaded with the differentiation stimulating factor of step (2) to a cell suspension and then dispensing them into a cell adhesion-inhibited microwell to prepare a cell spheroid; and
(4) obtaining an extracellular vesicle from the cell spheroid of step (3).

2. The method of claim 1, wherein the preparing of the microparticle of step (1) comprises: mixing a sugar solution and a polymer solution to obtain an emulsion solution; and adding the emulsion solution dropwise to a polyvinyl alcohol solution followed by stirring and then evaporating a solvent present in the solution.

3. The method of claim 1, wherein an average diameter of the microparticle in step (1) is 15 µm to 22 µm.

4. The method of claim 1, wherein the loading of step (2) comprises adding the microparticle of step (1) to a solution in an ionically bonded state having gelatin mixed in the differentiation stimulating factor and reacting them.

5. The method of claim 4, wherein the differentiation stimulating factor of step (2) is selected from one or more selected from the group consisting of TGF-β1, TGF-β3, BMP-2, BMP4, and bile acids.

6. The method of claim 1, wherein an average loading efficiency of the differentiation stimulating factor of step (2) is 50% to 60%.

7. The method of claim 1, wherein the cell suspension of step (3) is a cell suspension which is obtained from one or more selected from the group consisting of human adipose-derived mesenchymal stem cells, human bone marrow-derived mesenchymal stem cells, human hematopoietic stem cells, human chondrocytes, and human induced pluripotent stem cell-derived mesenchymal stem cells.

8. The method of claim 1, wherein an average diameter of the cell spheroid of step (3) is 75 µm to 115 µm.

9. The method of claim 8, wherein the cell spheroid has increased expression of COL2 and ACAN proteins.

10. The method of claim 1, wherein the extracellular vesicle of step (4) is obtained by adding a cartilage differentiation-inducible culture medium into the cell spheroid followed by culture.

11. The method of claim 10, wherein the extracellular vesicle is a cartilaginous functional extracellular vesicle.

12. A pharmaceutical composition for tissue regeneration, comprising the extracellular vesicle obtained by the production method according to any one of claims 1 to **11** as an active ingredient.

13. The pharmaceutical composition of claim 12, wherein the tissue regeneration is for regeneration of cartilage tissues.

14. A pharmaceutical composition for preventing or treating a cartilage disease, comprising the extracellular vesicle obtained by the production method according to any one of claims 1 to **11** as an active ingredient.

15. A health functional food composition for improving cartilage functions, comprising the extracellular vesicle obtained by the production method according to any one of claims 1 to 11 as an active ingredient.
